# EUROPÄISCHE PATENTANMELDUNG

(11) **EP 2 074 936 A1**
(43) Veröffentlichungstag der Anmeldung: **01.07.2009**
(21) Anmeldenummer: 08171492.5
(22) Anmeldetag: 12.12.2008
(51) Int. Cl.: A61B 1/267, A61B 5/08, A61D 9/00, A61B 1/005

(54) **Fixierbare Vorrichtung zur Untersuchung von Organen am Kopf eines Tieres**

(30) Priorität: 20.12.2007 DE 202007017840 U; 14.05.2008 DE 102008023554
(71) Anmelder: Dr. Fritz GmbH Endoskope und Videosysteme, 78532 Tuttlingen (DE)
(72) Erfinder: Fritz, Rolf, 78532 Tuttlingen (DE)
(74) Vertreter: Fitzner, Uwe

(57) **Zusammenfassung**

Die vorliegende Anmeldung betrifft eine Vorrichtung zur Untersuchung von Organen am Kopf eines Tieres mittels Sonden und Schläuchen **dadurch gekennzeichnet, dass** sie ein am Kopf des Tieres befestigbares Mittel (1) zur Fixierung der schlauchförmigen Teile und/oder Kabel der Vorrichtung aufweist.

## Beschreibung

Die vorliegende Erfindung betrifft eine Vorrichtung zum Untersuchen von Organen am Kopf von Tieren, insbesondere eines Pferdes mittels Sonden und Schläuchen.

Nach dem bisherigen Stand der Technik war es nur möglich, mittels Sonden und Schläuchen am stehenden Pferd Untersuchungen durchzuführen. Bestimmte Krankheiten treten aber nur während der Bewegung auf bzw. müssen während des Bewegungsablaufes des Pferdes untersucht werden. Hierzu zählen beispielsweise Kehlkopferkrankungen. Am bewegten Pferd waren jedoch Untersuchungen mittels Sonden und Schläuchen nur unter sehr erschwerten Umständen möglich. Ein Hauptgrund hierfür ist die bisher fehlende und unzureichende Fixierungsmöglichkeit am Kopf des Pferdes. Das Problem besteht sowohl bei der Bewegung unter dem Reiter als auch beim Traben und Galoppieren im freien Gelände oder in der Halle oder auch bei der Bewegung auf Laufbändern.

Aufgabe der vorliegenden Erfindung ist es nunmehr eine Vorrichtung zum Untersuchen von Organen auch am Kopf von Tieren, vorzugsweise Pferden mittels Sonden und Schläuchen zur Verfügung zu stellen, welche auch am bewegten Tier einsetzbar ist.

Diese Aufgabe wird durch ein am Kopf des Tieres, vorzugsweise des Pferdes befestigbares Mittel, auf welchem schlauchförmige Teile und/oder Kabel fixierbar sind, gelöst.

Als schlauchförmige Teile kommen erfindungsgemäß insbesondere Endoskope in Betracht.
Das erfindungsgemäße Mittel ist an jeder geeigneten Position am Kopf des Tieres anbringbar. Bei Pferden ist z. B. eine Lage zwischen den Ohren und Augen bis zu den Nüstern herabreichend bevorzugt. Möglicherweise ist aber auch eine seitliche Lage vorteilhaft, d. h. in der Achse vom Ohr- zur Maulspalte.

Das erfindungsgemäße Mittel ist anatomisch an dem Kopf des Tieres, insbesondere des Pferdes anpassbar. D. h. es besteht aus einem flexiblen biegbaren Material.

Daneben kann auch in Anpassung an dem Kopf des Pferdes das Mittel in der Länge verstellbar sein.

Erfindungsgemäß handelt es sich um ein bahnförmiges Gebilde, welches vorzugsweise zwischen den Augen verläuft und an seinem Ende in Höhe der Nüstern in deren Richtung biegbar ist, so dass ein hackenförmiges Gebilde entsteht.

Vorzugsweise ist das Mittel z. B. Bei Pferden am Halfter befestigbar. Die Verbindung auf dem Halfter kann beispielsweise mittels Klettverschlüssen erfolgen. Aber auch andere dem Fachmann geläufige Fixierungsmöglichkeiten kommen in Betracht. Vorzugsweise sollte jedoch eine Fixierung und auch Entfernung des Hakens möglich sein. Hierbei muss die Fixierung selbstverständlich so ausgestaltet sein, dass sie sich während der Bewegung des Pferdes nicht löst.

Das erfindungsgemäße Mittel ist in der Länge an den Kopf des Tieres, z. B. des Pferdes anpassbar. Ebenso ist eine Richtungsanpassung des Endes entsprechend der jeweiligen Nasenöffnung und der Kopfgröße möglich.

Das erfindungsgemäße Mittel ist vorzugsweise in Form einer Rinne ausgestaltet. In diese Rinne können die schlauchförmigen Teile der erfindungsgemäßen Untersuchungsvorrichtung eingelegt werden. Mittels geeigneter Vorrichtungen können die Schläuche fixiert werden. Hierzu zählen Klettverschlüsse, Bänder oder auch Schnallen. Ebenso sind aber auch andere dem Fachmann geläufige geeignete Methoden denkbar.

Das erfindungsgemäße Mittel kann aus beliebigem Material hergestellt werden. Bevorzugt ist jedoch Leder.

In einer erfindungsgemäß besonderen Ausgestaltung besteht das erfindungsgemäße Mittel aus zwei parallel laufenden Drähten. Diese sind biegbar, jedoch so ausgestaltet, dass nach der Biegung eine plastische Verformung bleibt. Diese plastische Verformung kann jedoch jederzeit rückgängig gemacht werden. D. h. , das Material muss für eine plastische Verformung ausgelegt sein, gleichwohl muss die plastische Verformung mittels mechanischer Mittel auch rückgängig zu machen sein.

Die parallel verlaufenden Drähte sind mit geeignetem Material umhüllt. Erfindungsgemäß kommt vorzugsweise Leder in Betracht. In einer besonders einfachen Ausführungsform sind die Drähte in Leder eingenäht und durch eine Mittelnaht voneinander getrennt. Die Mitteldraht bildet sodann eine Rinne, in welche Sonden oder Schläuche eingelegt werden können.

Durch zusätzliche Sicherungsmaßnahmen, beispielsweise einen Sicherungsriemen auf mittlerer Höhe der Nasen über der Sonde, dem Haken oder dem Halfter entsteht eine rutschfeste und stabile Verbindung zum Kopf des Tieres.

Das erfindungsgemäße Mittel hat den Vorteil, dass eine Sonde in einem knickgeschützten klar definierten Verlauf auf dem Nasenrücken des Pferdes angeordnet werden kann. Die Konstruktion erlaubt eine einfache Größenanpassung entsprechend der Kopfgröße und eine Anpassung der entsprechend den anatomischen Gegebenheiten der Nasenöffnung. Der Aufbau garantiert einen sicheren Sitz der eingelegten Sonde auch während der Bewegung des Tieres, d. h. sowohl in einer ruhigen Gangart als auch im gestreckten Galopp. Das System ist zudem einfach in der Herstellung und erlaubt eine individuelle Anpassung entsprechend den jeweiligen Erfordernissen.

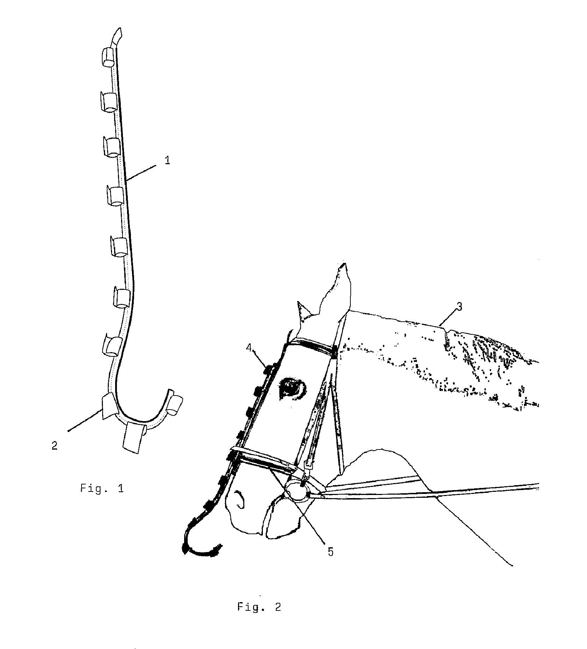

Die Erfindung wird im Folgenden anhand der Figuren beschrieben. In Figur 1 ist der erfindungsgemäße Haken dargestellt.

In Figur 2 ist die Anordnung auf dem Nasenrücken des Pferdes dargestellt.

Das in der Figur 1 dargestellte erfindungsgemäße Mittel in Form eines Hakens 1 ist in Richtung der Nüstern des Pferdes gebogen. Mittels der Schlaufen bzw. Bänder 2 kann ein schlauchförmiges Teil auf diesem Mittel 1 befestigt werden.

Gemäß Figur 2 ist der Haken 1 mit dem schlauchförmigen Teil der Untersuchungsvorrichtung auf dem Nasenrücken 4 des Pferdes 3 befestigt.

## Patentansprüche

1. Vorrichtung zur Untersuchung von Organen am Kopf eines Tieres mittels Sonden und Schläuchen **dadurch gekennzeichnet,**
**dass** sie ein am Kopf des Tieres befestigbares Mittel (1) zur Fixierung der schlauchförmigen Teile und/oder Kabel der Vorrichtung aufweist.

2. Vorrichtung nach Anspruch 1 **dadurch gekennzeichnet,**
**dass** die schlauchförmigen Teile flexible Endoskope sind.

3. Vorrichtung nach einem der vorhergehenden Ansprüche **dadurch gekennzeichnet,**
**dass** das Mittel (1) am Halfter (5) des Tierkopfes angeordnet ist.

4. Vorrichtung nach einem der vorhergehenden Ansprüche **dadurch gekennzeichnet,**
**dass** das Mittel (1) anatomisch an dem Kopf des Tieres anpassbar ist.

5. Vorrichtung nach einem der vorhergehenden Ansprüche **dadurch gekennzeichnet,**
**dass** das Mittel (1) in der Länge verstellbar ist.

6. Vorrichtung nach einem der vorhergehenden Ansprüche **dadurch gekennzeichnet,**
**dass** das Mittel (1) aus plastisch biegbarem Material besteht.

7. Vorrichtung nach einem der vorhergehenden Ansprüche **dadurch gekennzeichnet,**
**dass** das Mittel (1) aus Metalldrähten besteht, welche mit Leder umhüllt sind.

8. Vorrichtung nach einem der vorhergehenden Ansprüche **dadurch gekennzeichnet,**
**dass** das Mittel (1) aus zwei Metalldrähten besteht, welche mit Leder umhüllt sind und durch eine Mittelnaht voneinander getrennt sind.

9. Vorrichtung nach einem der vorhergehenden Ansprüche **dadurch gekennzeichnet,**
**dass** das Mittel (1) eine Rinne aufweist, welche die schlauchförmigen Teile aufnimmt.

10. Vorrichtung nach einem der vorhergehenden Ansprüche **dadurch gekennzeichnet,**
**dass** die Mittelnaht derart ausgestaltet ist, dass eine Rinne zur Aufnahme der schlauchförmigen Teile entsteht.
